# EUROPEAN PATENT APPLICATION

(11) **EP 4 841 328 A1**
(43) Date of publication of application: **18.11.2026**
(21) Application number: 25176945.1
(22) Date of filing: 16.05.2025
(51) Int. Cl.: G02B 21/24, G02B 21/00, G06T 7/55, A61B 5/00

(54) **DEVICE FOR ADJUSTMENT OF A FOCUS OF AN IMAGING DEVICE**

(71) Applicant: Leica Instruments (Singapore) Pte Ltd., Singapore 618299 (SG)
(72) Inventor: YANG, Gao, 618299 Singapore (SG)
(74) Representative: Paustian & Partner Patentanwälte mbB

(57) **Abstract**

A first aspect of the present disclosure is related to a device for adjustment of a focus of an imaging device,
configured to:
- obtain a first image of a sample, from an imaging device;
- obtain a second image of the sample;
- match at least a part of the first image to the second image and/or a part of the second image to the first image;
- determine a depth information for the matched parts of the first image and of the second image;
- determine a focus information based on the depth information;
- provide the focus information for adjustment of a focus.

## Description

### Technical Field

This disclosure is related to devices for adjustment of foci of imaging devices, such as microscopes, and to methods to operate such devices.

### Background

Traditional camera focusing may rely on a user's manual adjustment or on contrast-based autofocus algorithms. For contrast-based autofocus methods a camera captures successive frames while incrementally shifting lens position, computing each frame's image contrast, e.g. based on edge strength. Contrast increases as focus improves, peaks at best focus, then declines. The algorithm reverses direction whenever contrast drops, iteratively stepping toward the maximum until the peak is found and locked. However, in particular in cases, when an imaging device is far out of focus it is difficult, for a user or a machine, to understand in which direction a contrast-based focus of the image can be found. Improvements are desirable.

### Summary

An object of the present disclosure is to improve a focus adjustment of an imaging device

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a device for adjustment of a focus of an imaging device,
*configured to:*
- *obtain a first image of a sample, from an imaging device;*
- *obtain a second image of the sample;*
- *match at least a part of the first image to the second image and*/*or a part of the second image to the first image;*
- *determine a depth information for the matched parts of the first image and of the second image;*
- *determine a focus information based on the depth information;*
- *provide the focus information for adjustment of a focus.*

A device according to the first aspect (also "focus adjustment device" in the remainder) may be a control device, e.g. a specially programmed computer, configured to control an imaging device and/or it can be a part of the imaging device. A device may be an imaging device itself configured to observe a sample from two perspectives, in particular in a medical and/or diagnostical context. An imaging device can be, e.g., a microscope, an exoscope and/or a laparoscope. An imaging device may be configured to communicate with a device according to the first aspect of this disclosure. An imaging device can be a single view image imaging device or a stereo view imaging device. An imaging device may be configured to obtain at least a first image by its single view or stereo view camera, which can then be communicated to a device according to the first aspect. A second image can also be captured by the imaging device and communicated to the device in the same way. Additionally or alternatively, a second image can be obtained by a camera of a different device and afterwards obtained by the device according to the first aspect. A second image captures a sample from a different perspective as the first image.

A sample can be any object and/or specimen that can be viewed with an imaging device. In a surgical or diagnostical context, a sample can be an organ of a patient and/or a part of an organ. It can be a biopsy, in particular one that is isolated and analyzed in situ. A sample can be or comprise a bone and/or blood of a patient. A sample can also comprise a suture, an incision, or any other artifact placed within or at a being's body for or during a surgical procedure.

A focus adjustment device is configured to match at least partly the first image to the second image or vice versa. This can be done by a pixel-by-pixel search. Additionally or alternatively, this can be done by other image-matching methods, such as frequency-based methods, perceptual hashing, methods based on the Hamming distance, template or correlation matching, local feature pipelines, and/or methods based on artificial intelligence, such as neuronal networks. In a first image or in a second image, a region of interest (ROI) may be determined. The ROI may then be search for in the other of the two images, i.e. in the second image or in the first image. The focus adjustment may then be performed based on the matched ROIs.

For the matched images or parts of the images, a depth information can be determined. A depth information may be any kind of depth information which can be related to a stereo information comprised by the first and the second images of the sample. A depth information may be a depth map, which may be an image and/or representation where each pixel value encodes a distance (depth) from a camera sensor to a corresponding point in a scene and/or about a sample. A depth map may quantify the spatial structure of the environment captured in the images. Also a depth map may be determined based on the whole left and right images or based on a part of this images. Then this information is already available if a region of interest (ROI) is selected. On the other hand a depth information, such as disparity, may be computed only for a pair of matched ROIs in a first and a second image.

Based on depth information a focus information is determined. A focus information may be related to per-pixel sharpness and/or blur caused by being off the focal plane. Focus information may be related to a focus direction and/or a focus distance, i.e. how much a focus must be changed to get into focus. Focus information may be derived from a known camera model and/or a relationship between a first and a second image and depth information such as a depth map derived based on the first and the second image.

A focus information, in particular a focus direction and/a focus distance is derived based on depth information. Therefore, the imaging devices may be calibrated to the optomechanical system. This may be done such that the determined depth information corresponds to a relative focus offset or an absolute focus position. A particular depth may correspond to a zero position of a focus offset. A depth value larger than it can indicate a positive focus offset, and/or a smaller depth value may indicate a negative focus offset. The sign of the focus offset may correspond to a direction in which a focus should be changed, and/or a magnitude of a focus offset may correlate to a distance of focus change. The factor of correlation may depend on a magnification on a given imaging device.

Based on the focus information a focus of the imaging device can be adjusted additionally or alternatively to a manual or contrast-based focus adjustment.

An embodiment of the first aspect is related to a device for adjustment of a focus of an imaging device,
*configured to:*
- *adjust an autofocus based on the focus information.*

An autofocus can be any algorithmic procedure, based on a provided focus information, that automatically adjusts a camera's lens position and/or in software chooses the sharpest frame of an image series so that selected scene points appear sharp or at least sharper then before the autofocus was applied. An autofocus may comprise a hardware-based focus and/or a software-based focus. In a hardware-based focus an autofocus may drive a motor based on the provided focus information. A software-based focus relates to a digital post-processing, in which pixels or frames are selected based on the provided focus information.

An embodiment of the first aspect is related to a device for adjustment of a focus of an imaging device,
*configured to:*
- *provide an adjustment information based on the focus information to a user of a manual focus device.*

By a manual focus device a user directly sets the lens-to-sensor distance instead of relying only on autofocus. This can be done, e.g. with a manual focus knob, a focus ring, a touch bar, and/or a software-based graphical user interface. For using a manual focus efficiently an adjustment information, e.g., a focus direction and/or a focus distance can be displayed to a visually, auditively, and/or haptically. For example direction can be shown to a user by an arrow on a display, providing the user with the information in which direction she/he should move a focus knob or a focus slider in order to reach the next focus. Additionally or alternatively, a size of the displayed arrow can show the user how much she/he must manipulated the focus control means in order to reach the focus. In this way a plurality of identified foci based on a plurality of identified sets of focus information can be addressed.

An embodiment of the first aspect is related to a device for adjustment of a focus of an imaging device,
*wherein the depth information comprises disparity information.*

A disparity information may be related to one or more disparity values for one or more pixels that are matched between a first image and a second image of a device according to the first aspect of this disclosure. A disparity information may comprise a disparity map. A disparity map may be a two-dimensional representation indicating, e.g., for each pixel in a stereo image, the difference in positions between a left and right image. Each pixel's intensity/value corresponds to a disparity, allowing recovery of relative depths and enabling three-dimensional understanding of a scene from the two-dimensional left and right images. Based on a disparity (map), pixels for any perspective on the baseline (i.e. a straight line between the left and the right image) can be determined. A disparity map can be determined by mapping a first image, e.g. a left image, to a second image, e.g. a right image, or vice versa. Additionally or alternatively, an set of corresponding ROIs is determined in a first and a second image, and a disparity information, e.g. a disparity map, is determined for the matched ROIs. Disparity maps can be created for stereo images of an imaging device. Since the stereo cameras may be aligned vertically, the matching problem, i.e. to find corresponding sample pixel of the image of the left camera in the right camera image, can be solved line by line.

For example, by calibration at a given magnification, a pair of dual perspective images (e.g. an image pair of a stereo camera and/or an image pair of two different camera capturing the same sample) may have an N-pixel disparity when in focus and a positive shift in disparity may require a focus to move closer to compensate. In the same situation, the factor of focus per pixel disparity may be F mm at the current focus position. In case M pixels of disparity is measured, it can be determined that X = (M-N)*F is the focus shift needed. Hence, the focus may move by abs(X) mm and/or the direction may depend on sign(X). This relationship may not be linear throughout the focus range. Therefore, a calculation may be repeated as focus is adjusted and in particular when one or more other parameters change, until the focus converges.

An embodiment of the first aspect is related to a device for adjustment of a focus of an imaging device,
*wherein the second image is from a further imaging device.*

A further imaging device may be an external camera. A further imaging device may also be an internal camera that does not directly contribute to an image captured by an imaging device, e.g. an overview camera. A further imaging device may be a single view camera or a stereo view camera.

An embodiment of the first aspect is related to a device for adjustment of a focus of an imaging device,
*wherein the first image is a left image of a stereo camera of the imaging device and the second image is a right image of the stereo camera of the imaging device.*

A left image and a right image that provided stereo information can be obtained from a stereo camera, in particular with a pair of images captured simultaneously by two slightly offset cameras. Additionally or alternatively, a single camera may be used to capture a left and a right image sequentially, moving the camera slightly between shots. Additionally or alternatively, synthetic left and right images may be generated computationally from a single image using depth-information obtained by other means. A left and a right image may be captured from a fixed viewpoint while varying the lighting direction of the sample. Additionally or alternatively, structured light techniques may be used to obtain a left and a right image from a single image of a sample. Additionally or alternatively, a camera paired with a depth sensor (e.g., LiDAR or time-of-flight camera) may combine an image with depth data to obtain stereo-like information on which a left image and a right image can be based on.

An embodiment of the first aspect is related to a device for adjustment of a focus of an imaging device,
*wherein the second camera image is obtained from an overview camera*.

An overview camera can be any camera that captures a sample from a different perspective than the camera that captures the first camera image of the imaging device. The overview camera can thereby be part of the imaging device. Additionally or alternatively, an overview camera may be an external camera that is configured to communicate its images to a focus adjustment device.

If a first and a second image is provided by two different cameras, a geometric relationship between the two cameras may be determined beforehand, in particular in order to determine a matching of the two image and/or in order to determined depth information. Therefore, one or both of the images may be processed in order to be compared with each other. For example, camera of the imaging device may be related to a main camera, i.e. a microscope camera. An overview camera may be related to a side camera. Additionally or alternatively, an overview camera may have a smaller resolution and/or related to a different field of view of a sample. Images of both cameras may be rescaled to match a magnification that the pixel data can be compared in order to match the image contents, in particular the captured parts of a sample. For example, a high resolution first image of a microscope camera may be downsized to match the resolution of a second image of an overview camera. Additionally or alternatively, a second image of an overview camera may be cropped to the size of the first image of an imaging device, in particular of a microscope. The adaptation of the two different images to each other may facilitate determination of matching image content and/or determination of depth information within the two images.

An embodiment of the first aspect is related to a device for adjustment of a focus of an imaging device,
*configured to:*
- *obtain an information about a region of interest in one of the two images* ; *and wherein the matching is performed based on the region of interest.*

A region of interest (ROI) may be a subset of a first image and/or of a second image that matters for analyzing a sample. An ROI may be described by a bounding box, by a pixel-wise mask, by polygons, by one or more contours for irregular shapes, and/or by sparse key points marking landmarks. An ROI can be chosen manually by a user, e.g. over a graphical user interface. Additionally or alternatively, an ROI may be automatically determined, e.g., with heuristics such as a center crop, by algorithmic object detectors, and/or via a machine learning algorithm such as a neural-network. An ROI may to be present, at least partly, in the first and in the second image in order to perform a matching of image content and/or of determining depth information. Additionally or alternatively, a user-defined ROI can be shifted based on calibration to remain at the same position relative to a scene, as the field of view may shift, in particular during focusing.

An embodiment of the first aspect is related to a device for adjustment of a focus of an imaging device,
*configured to:*
- *determine a defocus of the imaging device based on the first image* ; *and*
- *determine the focus information in* case *the defocus exceeds a pre-defined threshold.*

A defocus may relate to any metrics that qualifies an/or quantifies a state in which the imaging device is not in focus for a specific image. A defocus may, e.g., be based on a frequency and/or contrast measurement, e.g. by measuring how much high-frequency detail and/or edge sharpness an image lacks, e.g. as an absolute value and/or as a relative value, e.g., in comparison to a reference image of a similar sample and/or of a previously captured image. Additionally or alternatively, defocus determination can be based on blur metrics.

In case a defocus of an image is above a certain threshold it may be assumed that the respective image is largely out of focus. In this case a focus adaptation according to the first aspect of the present disclosure can be conducted, which may provide a direction to a focus and/or a distance for an adjustment means to reach the respective focus. After the focus adaptation is applied a focus of the image may not be a perfect focus. In this case a further focus may be used that may use in particular a focus direction determined by the focus adaptation.

An embodiment of the first aspect is related to a device for adjustment of a focus of an imaging device,
*configured to:*
- *determine a confidence score based on a plurality of matchings of at least a part of the first image to the second image and*/*or a part of the second image to the first image.*

A confidence score may inform a focus adaptation device how good a determined and/or provided focus information is. A confidence score may be determined based on a plurality of ROI matchings, in particular within a search of a best ROI matching between a first and a second image. A confidence score may be helpful to decide whether a focus adaptation of a focus adaptation device is used or whether another focus method is used to adapt the focus. If the confidence score is low, a user may be informed that the focus adaptation device is not confident enough to perform a focus adaptation. On the other hand, if a confidence score is high enough a user may be informed that she/he can use a provided focus information and/or the focus adaptation device may perform a focus adaptation based on the determined/provided focus information. This may prevent to conduct unnecessary movements of the lens system of the imaging device. A low confidence score may also be used to assess that a selected ROI is not usable because it does not contain sufficient details to be focused on. In this case another ROI could be selected automatically and/or a user may be prompted to select another ROI.

An embodiment of the first aspect is related to a device for adjustment of a focus of an imaging device,
*configured to:*
- *determine that a focus of the imaging device has been adjusted based on the focus information; and*
- *determine an additional focus based on the adjusted focus.*

The focus adaptation of a focus adaptation device may be used as a coarse focus, in particular if it is determined that an image is out of focus over a pre-defined threshold. Then the focus adaptation can be performed to provide a focus direction in which the focus (or a subsequent focus) should be adapted. The focus adaptation may be performed until no improvement can be determined based on the depth-bath focus adaptation. Afterwards, a different focus method can be deployed for a fine tuning, such as a contrast- and/or frequency-based focus.

An embodiment of the first aspect is related to a device for adjustment of a focus of an imaging device,
*configured to:*
- *decide if the focus information is determined or if another focus is used, based on the confidence score.*

A depth-based focus adaptation of a focus adaptation device may be performed depending on a confidence score value. If the confidence score value above a certain, pre-defined threshold, the depth-based focus adaptation may be performed. Otherwise another focus adaptation may be performed, such as a contrast- and/or frequency-based focus. In this case, the focus adaptation device may be configured to provide an output information based on which an imaging device may know that another focus needs to be selected. If the focus adaptation device also acts as a control device of the imaging device, this may not be necessary, and the selection of another focus may be done by the focus adaptation device itself.

An embodiment of the first aspect is related to a device for adjustment of a focus of an imaging device,
*configured to:*
*adjust one or both of the following parameters of a camera of the imaging device before a matching of the left and right images is performed:*
- *decrease magnification, in particular to a minimum;*
- *decrease aperture, in particular to a minimum.*

When the imaging device is out of focus, a change of magnification of the image, in particular such that a selected or determined ROI is comprised, may unveil structures that may be used for a focus adaptation. A magnification can be decreased, in particular to a minimum magnification that is possible for the imaging system. An additional post-processing to unveil potential image structures may also be conducted. Additionally or alternatively, decreasing an aperture, in particular to an adjustable minimum, may also reveal structures for a focus adaptation, in particular for a matching procedure. After the focus adjustment has been performed, the initial values may be restored, in particular an initial magnification of the first and/or the second image and/or an initial aperture for the first and the second image.

An embodiment of the first aspect is related to a device for adjustment of a focus of an imaging device,
*configured to:*
- *obtain a region of interest within the first or the second image;*
- *adapt the region of interest based on one or more:*
   - *the respective other of the first or the second image;*
   - *a newly determined field of view.*

Sometimes it may be helpful to change a magnification on one of a first or a second image during a focus adaption, e.g., to adapt the images to each other in order to perform a matching. In this case, an ROI selected prior to the procedure may have to be changed as well. For example, in case a first image has to be magnified in order to have the same size as a second image, a selected ROI or an ROI search candidate in the first image may have to be increased as well in order to have the same size as the ROI used for the second image. Additionally or alternatively, a position of the ROI within a first or a second image may be changed, based on a position of the respective other image and/or based on a newly determined field-of-view, in particular a newly obtained gaze direction by a user.

A second aspect of the present disclosure is related to a method for adjustment of a focus of an imaging device,
*comprising:*
- *obtaining a first camera image of a sample from an imaging device;*
- *obtaining a second camera image of a sample;*
- *matching at least a part of the first camera image to the second camera image or vice versa;*
- *determining a depth information for the matched part of the first image and of the second image;*
- *determining a focus information comprising a focus direction and*/*or a focus distance based on the depth information;*
- *providing the focus information for adjustment of a focus.*

A method according to the second aspect can comprise one or more steps to execute one or more functions of a focus adaptation device and/or of an imaging device according to the first aspect of this disclosure. Steps of a method according to the second aspect of this disclosure can be configured such that they comprise features of any of the aforementioned devices.

A further aspect of the present disclosure is related to a computing device comprising a processor configured to carry out the method according to any one of preceding aspects/embodiments.

A further process of the present disclosure is related to a computer program product comprising instructions which, when the program is executed by a computer system, cause the computer system to carry out the method according to any one of the preceding aspects/embodiments.

A further aspect of the present disclosure is related to a computer-readable medium comprising instructions which, when executed by a computer system, cause the computer system to carry out the method according to any one of the preceding aspects/embodiments.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a device according to an embodiment of this disclosure.
Fig. 2 illustrates a device according to an embodiment of this disclosure.
Fig. 3 illustrates a device according to an embodiment of this disclosure.
Fig. 4 illustrates steps within foci adjustments according to two embodiments of this disclosure.
Fig. 5 illustrates steps within foci adjustments according to two embodiments of this disclosure.
Fig. 6 illustrates a microscope system according to or for embodiments of this disclosure.

Although some aspects have been described in the context of an apparatus (or a system) in the present disclosure, the description of these aspects also represents a description of the corresponding method, where a block or device corresponds to a method step or a feature of a method step.

Analogously, aspects described in the context of a method step also represent a description of a corresponding block, item, or feature of a corresponding apparatus or of a system that may in particular be distributed over different locations and is configured to exchange information between the different locations with respective communication means.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the aspects or embodiments of this disclosure, until explicitly stated otherwise.

In the following description reference is made to the accompanying figures which form part of the disclosure, and which illustrate specific aspects in which the present disclosure can be understood. Identical reference signs refer to identical or at least functionally or structurally similar features.

### Detailed description

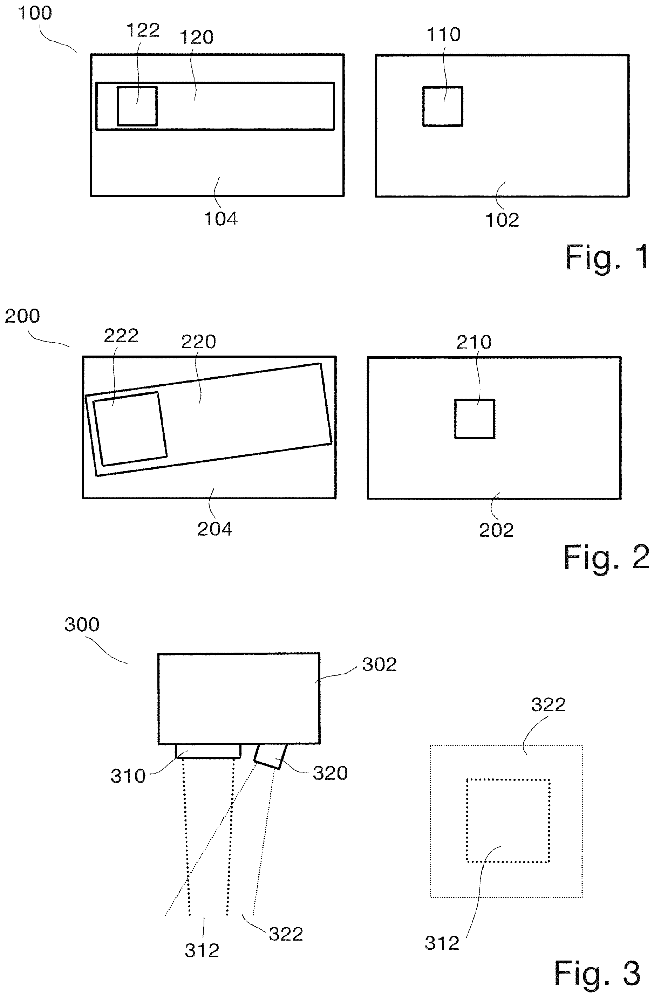

Fig. 1 illustrates a focus adjustment device 100 according to an embodiment of this disclosure. In this case a stereo camera of an imaging device is used for determining a focus information. The figure shows two images, a right image 102 and a left image 104 of a stereo camera view. A region of interest (ROI) 110 is defined in the right image 102. This can be done automatically and/or based on a user input, e.g. a user selection. After the ROI 110 has been defined, the focus adjustment device 100 searches for a corresponding ROI 122 in the left stereo image 104. This can be done be a comparison of structural and/or chromatic features, for example. Therefore, a plurality of candidate ROIs can be identified. Since the stereo camera captures right images 102 and left images 104 on the same height relative to the sample, the search for the selected ROI 110 in the left image can be conducted for the pixel height 120 in which the ROI110 in the right image was selected. Hence the left image ROI is searched in the frame 120. Eventually, the left image ROI 112 is determined as the pixel field with the same size and with the smallest deviations of the ROI 110 of the right image 102. Based on the differences to the other ROI candidates a confidence value can be determined that provides information on how good the estimate is. If the best estimate is deemed to be good enough (e.g. by its confidence score exceeding a pre-defined threshold) the matching of the ROIs in both images is concluded.

Based on the matched ROIs a disparity map for each pixel in the ROIs can be determined. An based on the disparity map a focus information is determined. The focus information comprises the direction in which a focus position can be found. Additionally the focus information comprises a distance to the suggested focus position. Based on the depth information the focus information can be determined based on the following relationship. In stereo image processing, focus is proportional to depth information. Hence, the higher an identified depth of a structure within an image, the larger a focus has to be adapted. Additionally and alternatively, focus is inversely related to disparity. Hence, the larger an identified disparity for a certain pixel, the smaller the focus must be. After the focus information is determined based on depth information (which comprises inversely related disparity information), the focus information is provided to the imaging device in order to adjust its focus system for a sharp image.

For example, by calibration at a given magnification, a stereoscopic pair may have a 5-pixel disparity when in focus and a positive shift in disparity may require a focus to move closer to compensate. In the same situation, the factor of focus per pixel disparity may be 0.2 mm at the current focus position. In case -20 pixels of disparity is measured, it can be determined that -20 - 5 = - 25, then -25 * 0.2 = -5. Hence, the focus moves by 5 mm further away. The calculation may be repeated as focus is adjusted and in particular when one or more other parameters change. The adjustment can be terminated in case the focus has converged.

Fig. 2 illustrates a focus adjustment device 200 according to an embodiment of this disclosure. In this case, an image of an imaging device is combined with an image of an external camera in order to determine a focus information. The figure shows two images, a right image 202 and a left image 204. The right image 202 is from an external camera that is not aligned with the camera of the imaging device that captures image 104. Therefore, an ROI 210 defined in the right image 202 has a different size compared to an ROI 222 in the left image. Also the area 220 in which a matching ROI 222 to an initially selected/determined ROI 210 is searched, has an orientation that is not parallel to one of the edges of the image 202. Therefore, the focus adjustment device may be configured to communicate with the external camera to obtain position information, in particular orientation information of the images captured by the external camera, and information about the field-of-view of the external camera. Furthermore, also resolution information may be obtained. Based on this information, the orientation of the search area 220 can be determined within the first image (i.e. the image of the imaging device) relative to the second image of the external camera. Additionally, based on the image size information, the size of the ROI 210 selected or defined in the external camera image can be adapted to a size of ROI search candidates 222 within the image of the image device. After a matching of the ROIs has been concluded, depth information (e.g. disparity information) can be determined based on the two different perspectives captured by the left image (of the image device) and of the right image (of the external camera). A focus information can then be determined in the same way as described for the previous figure.

Fig. 3 illustrates a microscope 300, which on the same time is a focus adjustment device according to an embodiment of this disclosure. In this embodiment, the stereo information for determining a focus information is derived from a main surgeon camera 310 and from an assistant camera or side view or overview camera 320. Both cameras are housed in the optical system REF302 of the microscope 300. The main surgeon camera captures an image with a first field-of-view (FOV) 312. The assistant or side view camera captures an image from another perspective and with a second FOV 322. Since the first FOV differs from the second FOV the images of the assistant camera 310, 320 are cropped to match the FOV of the images of the main surgeon camera 312. Furthermore, the main surgeon camera 310 has a much higher resolution than the assistant camera 320, which is rather an overview camera with a simpler optical system. Therefore, the resolution of images by the main surgeon 310 may be adapted to match the resolution of the assistant camera 320. ROIs can be selected either in the view of main surgeon camera 310 or in the view of the assistant camera. Since the assistant camera has a larger FOV for manual selection of an ROI in the view 322 of the assistant camera, the FOV 312 of the main surgeon camera is indicated (by the dotted rectangle, in the right subimage) in the FOV 322 of the assistant camera 320. Based on the adjusted images of both cameras, ROI matching can be performed, depth information can be determined and based on the depth information a focus information for focus adjustment is determined.

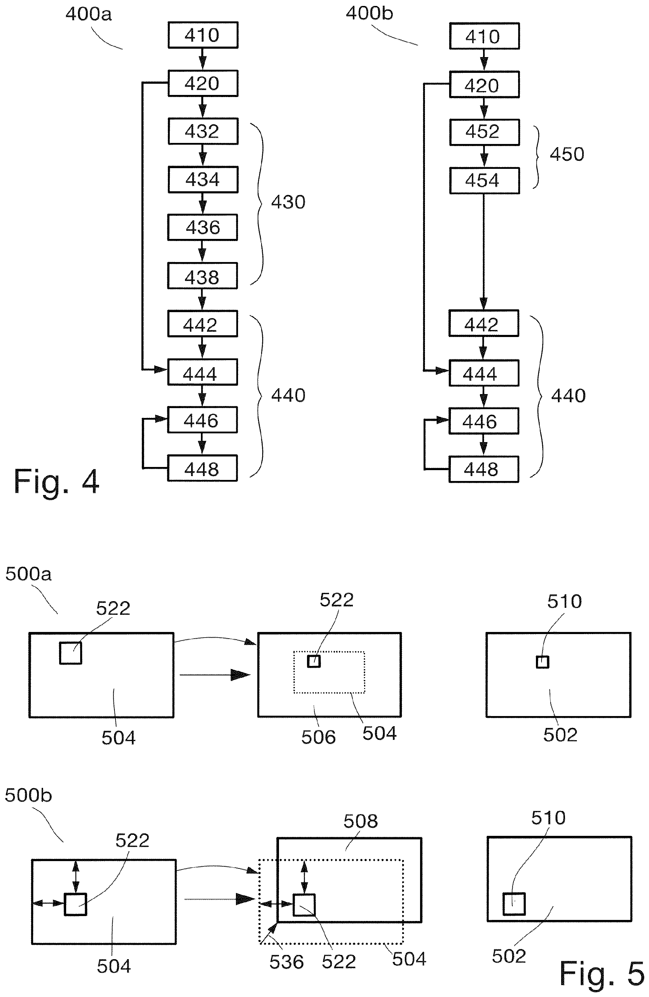

Fig. 4 illustrates steps within focus adjustments according to two embodiments of this disclosure. In this case a microscope with a stereo camera that provides a right image and a left image and/or an overview image a of a sample may be used.

The flowchart 400a on the left side starts with a selection of an ROI 410 within one of the images. This can be done by a user. Additionally or alternatively, an ROI can be suggested and/or selected by an ROI-identification procedure. For example, an ROI can be selected based on a visual gaze of a surgeon. Therefore, the gaze must be measured which can be done, e.g., by an eye tracker. Based on the provided ROI a stereo matching 420 is performed between a left and a right image of the stereo camera. In case the defocus increases a pre-defined threshold it may be decided that a contrast-based focus is not suitable and a coarse focus 430 based on a depth-based focus adjustment will be performed. The coarse autofocus starts by setting the magnification and/or the aperture of the stereo camera to their respective minimums 432. This increases a depth-of-field of the observed image and more structures (i.e. image content) may become visible. Based on the new configuration a further stereo matching 434 may be performed. Based on the best matching a depth information, e.g. a disparity map may be determined and based on that a depth based focus information can be determined. The depth based focus information may comprise a focus direction as well as a focus distance. Based on this information a coarse autofocus is performed 436. After the coarse autofocus has been completed the initial settings of magnification and/or aperture are adjusted again 438. After the coarse autofocus has been finished a contrast-based autofocus 440 may be performed as a fine tuning of the already adjusted autofocus 430. A contrast-based autofocus may start with a matching of a selected ROI in one of the images of the stereo camera to the other of the two images of the stereo camera. This can be the same ROI as previously selected. Alternatively, a new ROI may be selected, and a new matching may be performed 442. The fine autofocus 440 is realized based on the newly matched ROI. Based on the matched ROIs an autofocus can be adjusted 444. Afterwards a contrast can be determined 446 for the ROI and potentially the autofocus can be adjusted further 448. This can be repeated, e.g., as long as a contrast improvement can be observed.

The flowchart 400b on the right side also starts with a selection of an ROI 410 within one of the images and an initial stereo matching 420. In this embodiment no change of magnification and/or aperture is performed. The coarse focus 450 is therefore simpler as in the embodiment which is described by the flowchart on the left. It comprises a step 452 in which a matching of a left image to a right image is performed. In case both images are not captured by the same camera (e.g. the left image is captured by a main microscope camera and the right image is captured by an overview camera) a size and/or perspective-based correction may be performed before a depth information is determined. Then depth-based focus information is determined and an autofocus is adjusted 454. Afterwards a fine autofocus 440 can be run again in order to further sharpen the observed images.

Fig. 5 illustrates steps within foci adjustments according to two embodiments of this disclosure. The upper subfigure 500a illustrates a first ROI adaptation, in which a ROI is changed in size for a matching procedure. The subfigure 500a shows a first image 502, which is an image of a camera external to an imaging device and which shall be used for a focus adaptation. The external camera is configured to observe the same sample as the camera of the imaging device, but from another perspective. This allows a focus adjustment based on depth information. Therefore, an ROI 522 is selected in an image of the imaging camera. The external camera has provided information about its FOV to the imaging device. Based on this information the imaging device is configured to adapt the second image 504. In this case the image has to be resized to the virtual image 506 such that the ROI522 has the same size as the ROI in the first image 502. Based on this adaptation a matching of the ROI of the second image (image of the imaging device) in the first image 502 can be performed. The search for a matching ROI 510 should remain in the area of the first image (dotted line 504 in virtual image 506). Only in this area a matching can be found, because only this area is captured by the first image (image of the imaging device) and the second image (image of the external camera) on the same time.

The lower subfigure 500b a second ROI adaptation, in which a ROI is changed in position for a matching procedure. The subfigure 500b shows a first image 502, which is an image of a camera external to an imaging device and which shall be used for a focus adaptation. As in the previous example, the external camera is configured to observe the same sample as the camera of the imaging device, but from another perspective. Therefore, an ROI 522 is selected in an image of the imaging camera. The external camera has provided information about its FOV to the imaging device. Based on this information the imaging device is configured to adapt the first image 504. In this case, the image 504 is virtually shifted to another position in order to allow matching of the ROI 522 in the image 502 of the external camera. The image 504 is virtually shifted to the virtual image 508 based on the vector 536. After the shift, the ROI selected in the first image has a different position in the virtual image 508. In this position in can be matched horizontally with the image 502 of the external camera. In this case, the search should remain in the area defined by the intersection of the original image 504 and the virtual image 508. Only in this area a matching can be found, because only this area is captured by the first image (image of the imaging device) and the second image (image of the external camera) on the same time.

For both described embodiments it is also possible that the camera of the imaging device captures the first image 502 and an external camera captures the image 504. The above explanations then hold mutatis mutandis.

Additionally or alternatively, the lower subfigure 500b may be related to a stereo camera view, which is shifted by the vector 536. The middle image 504 and the left image 502 of the subimage represent the shifted left and right image of the stereo camera. The left image 504 of the subimage represents the left image of the stereo camera before the field of view was shifted. As it can be seen by a comparison of the old and the new image 504, in particular as the focus is adjusted, a field of view may shift by the vector 536, and the ROI 522 needs to remain stationary relative to the scene 508. Therefore, the ROI needs to be shifted to a different position 522, 510 in the new frames 508, 502.

Some embodiments relate to a microscope comprising a system as described in connection with one or more of the Figs. 1 to 5. Alternatively, a microscope may be part of or connected to a system as described in connection with one or more of the Figs. 1 to 5.

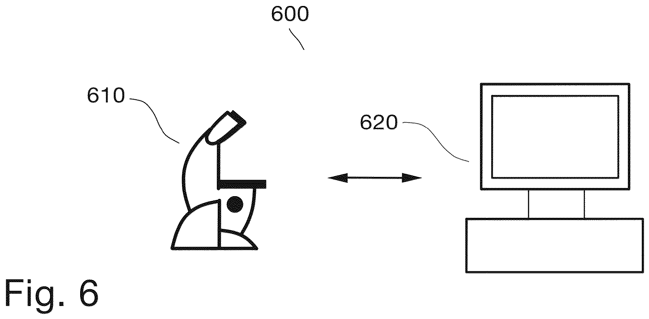

Fig. 6 shows a schematic illustration of a system 600 configured to perform a method described herein. The system 600 comprises a microscope 610 and a computer system 620. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 3. Alternatively, a microscope may be part of or connected to a system as described in connection with one or more of the Figs. 1 to 3. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference signs

- 100: device for focus adjustment
- 102: right image
- 104: left image
- 110: region of interest
- 120: search frame
- 122: corresponding ROI
- 200: focus adjustment device
- 202: right image
- 204: left image
- 210: region of interest
- 220: ROI search area
- 222: corresponding ROI
- 300: microscope
- 302: optical system
- 310: main surgeon camera
- 312: FOV main surgeon camera
- 320: overview camera
- 322: FOV assistant camera
- 400a: focus adjustment
- 400b: focus adjustment
- 410: select ROI
- 420: stereo matching
- 430: coarse AF
- 432: set minimum magnification and minimum aperture
- 434: determine stereo matching
- 436: drive AF
- 438: configure previous magnification and aperture
- 440: fine AF
- 442: perform stereo matching
- 444: drive AF
- 446: perform contrast detection
- 448: drive AF
- 450: coarse AF
- 452: determine stereo matching or AF
- 454: drive AF
- 500a: first ROI adaptation
- 500b: second ROI adaptation
- 502: first image
- 504: second image
- 506: virtual image
- 508: virtual image
- 510: corresponding ROI
- 522: ROI
- 536: shifting vector

## Claims

1. A device for adjustment of a focus of an imaging device,
configured to:
- obtain a first image (102) of a sample, from an imaging device;
- obtain a second image (104) of the sample;
- match at least a part of the first image to the second image and/or a part of the second image to the first image;
- determine a depth information for the matched parts of the first image and of the second image;
- determine a focus information based on the depth information;
- provide the focus information for adjustment of a focus.

2. The device according to the preceding claim,
configured to:
- adjust an autofocus based on the focus information.

3. The device according to one of the preceding claims,
configured to:
- provide an adjustment information based on the focus information to a user of a manual focus device.

4. The device according to one of the preceding claims,
wherein the depth information comprises disparity information.

5. The device according to one of the preceding claims,
wherein the second image is from a further imaging device (320).

6. The device according to one of the preceding claims,
wherein the first image (102) is a left image of a stereo camera (310) of the imaging device and the second image (104) is a right image of the stereo camera of the imaging device.

7. The device according to one of the preceding claims,
wherein the second camera image (322) is obtained from an overview camera (320).

8. The device according to one of the preceding claims,
configured to:
- obtain an information about a region of interest (110, 120) in one of the two images (102, 104); and
wherein the matching is performed based on the region of interest.

9. The device according to one of the preceding claims,
configured to:
- determine a defocus of the imaging device based on the first image (104);
and
- determine the focus information in case the defocus exceeds a pre-defined threshold.

10. The device according to one of the preceding claims,
configured to:
- determine a confidence score based on a plurality of matchings of at least a part of the first image to the second image and/or a part of the second image to the first image.

11. The device according to one of the preceding claims,
configured to:
- determine that a focus of the imaging device has been adjusted based on the focus information; and
- determine an additional focus based on the adjusted focus.

12. The device according to one of the preceding two claims,
configured to:
- decide if the focus information is determined or if another focus is used, based on the confidence score.

13. The device according to one of the preceding claims,
configured to:
adjust one or both of the following parameters of a camera of the imaging device before a matching of the left and right images is performed:
- decrease magnification, in particular to a minimum;
- decrease aperture, in particular to a minimum.

14. The device according to one of the preceding claims,
configured to:
- obtain a region of interest within the first or the second image;
- adapt the region of interest based on one or more:
- - the respective other of the first or the second image;
- - a newly determined field of view.

15. A method for adjustment of a focus of an imaging device,
comprising:
- obtaining a first camera image of a sample from an imaging device;
- obtaining a second camera image of a sample;
- matching at least a part of the first camera image to the second camera image or vice versa;
- determining a depth information for the matched part of the first image and of the second image;
- determining a focus information comprising a focus direction and/or a focus distance based on the depth information;
- providing the focus information for adjustment of a focus.
